# EUROPÄISCHE PATENTANMELDUNG

(11) **EP 1 604 644 A1**
(43) Veröffentlichungstag der Anmeldung: **14.12.2005**
(21) Anmeldenummer: 05005102.8
(22) Anmeldetag: 09.03.2005
(51) Int. Cl.: A61K 7/42, A61K 7/035

(54) **Emulsionskonzentrat mit wasserlöslichen und öllöslichen Polymeren**

(30) Priorität: 24.05.2004 DE 102004025357
(71) Anmelder: Beiersdorf AG, 20253 Hamburg (DE)
(72) Erfinder: Daniels, Rolf, Prof. Dr., 38226 Salzgitter (DE); Mundt, Claudia, Dr., 28207 Bremen (DE); Pfeifer, Svenja, 22301 Hamburg (DE)

(57) **Zusammenfassung**

Herstellung und Verwendung eines Emulsionskonzentrates erhältlich durch Trocknung einer O/W-Emulsion enthaltend
- eine Lipidphase,
- ein oder mehrere öllösliche Polymere;
- eine wässrige Phase,
- ein oder mehrere wasserlösliche Polymere,
- ein oder mehrere UV-Lichtschutzfilter und
gegebenenfalls weiteren kosmetischen und/oder dermatologischen Wirk-, Hilfs- und Zusatzstoffen, mit einem Wasseranteil von 0,1 bis 20 Gew.-%, bezogen auf das Gesamtgewicht der getrockneten Zubereitung.

## Beschreibung

Die vorliegende Erfindung betrifft ein pulverförmiges Emulsionskonzentrat und Verfahren zur Herstellung eines Emulsionskonzentrates, dadurch gekennzeichnet, dass eine O/W-Emulsion enthaltend
a) eine Lipidphase in einer Konzentration von 10 bis 85 Gew.-%,
b) ein oder mehrere wasserlösliche Polymere in einer Gesamtkonzentration von 1 bis 80 Gew.-%,
c) ein oder mehrere öllösliche Polymere in einer Gesamtkonzentration von 0,1 bis 50 Gew.-%,
jeweils bezogen auf das Gesamtgewicht der Zubereitung, sowie
d) ein oder mehrere UV-Lichtschutzfilter und
e) eine wässrige Phase,
neben gegebenenfalls weiteren kosmetischen und/oder dermatologischen Wirk-, Hilfs-und Zusatzstoffen, durch Walzen-, Kanal-, Sprüh- und/oder Gefriertrocknung entwässert bzw. getrocknet wird.

Das getrocknete, pulverförmige Emulsionskonzentrat kann mit verschiedenen Hilfsstoffen abgemischt werden, um ein verkehrsfähiges Endprodukt zu bilden.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut und der Hautanhangsgebilde, d.h. der Haare und der Nägel.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe und der UV-Strahlung. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte, in der Regel Cremes, Salben oder Lotionen, dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte bestehen in der Regel aus Emulsionen. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltern entwickelt, die in kosmetischen Zubereitungen eingesetzt werden können. Diese UVA- und UVB-Filter sind in den meisten Industrieländern in Form von Positivlisten wie der Anlage 7 der Kosmetikverordnung zusammengefasst.

Herkömmliche kosmetische und/oder dermatologische Emulsionen, beispielsweise Sonnenschutzcremes oder -Iotionen, haben aufgrund Ihres Wassergehaltes eine Reihe von Nachteilen:
■ Sie besitzen ein hohes Gewicht, was beim Transport zu höherem Energieverbrauch und höheren Kosten führt.
■ Sie besitzen ein größeres Volumen, was beim Transport zu geringeren Transportkapazitäten führt.
■ Insbesondere dünnflüssige Emulsionen sind wesentlich aufwendiger zu lagern und transportieren, da die Zubereitungen "auslaufen" können.
■ Die Anwendungskonzentrationen der Inhaltsstoffe (z.B. UV-Filterkonzentration und damit UV-Filterleistung) sind dem Verbraucher bereits vorgegeben. Eine individuelle Anpassung an die Gegebenheiten am Anwendungsort sind nicht mehr möglich.

Die Trocknung, d. h. der Entzug von Wasser, von Emulsionen ist bereits bekannt. Wird jedoch einer Emulsion die äußere Phase entzogen, so nähern sich die Tröpfchen der inneren Phase einander an und eine Koaleszenz wird begünstigt. Dies führt unweigerlich zum Brechen, das bedeutet zur Phasentrennung, der Emulsion.

Es war daher die Aufgabe der vorliegenden Erfindung, die Nachteile des Standes der Technik zu beseitigen und eine "trockene", stabile Emulsion sowie ein Verfahren zur Herstellung derselben zu entwickeln.

Überraschend gelöst wird die Aufgabe durch ein Verfahren zur Herstellung einer O/W-Emulsion, dadurch gekennzeichnet, dass eine O/W-Emulsion enthaltend
a) eine Lipidphase in einer Konzentration von 10 bis 85 Gew.-%,
b) ein oder mehrere wasserlösliche Polymere in einer Gesamtkonzentration von 1 bis 80 Gew.-%,
c) ein oder mehrere öllösliche Polymere in einer Gesamtkonzentration von 0,1 bis 50 Gew.-%,
jeweils bezogen auf das Gesamtgewicht der Zubereitung, sowie
d) in oder mehrere UV-Lichtschutzfilter und
e) eine wässrige Phase, neben gegebenenfalls weiteren kosmetischen und/oder dermatologischen Wirk-, Hilfs-und Zusatzstoffen, durch Walzen-, Kanal-, Sprüh- und/oder Gefriertrocknung entwässert bzw. getrocknet wird.

Unter entwässern bzw. trocknen ist im Sinne der Erfindung der Entzug von flüchtigen Bestandteilen der Emulsion zu verstehen, wobei ein festes bzw. wachsartiges Produkt entsteht.

Dabei ist es erfindungsgemäß bevorzugt, wenn die Lipidphase in einer Konzentration von 5 bis 85 Gew.-%, besonders bevorzugt in einer Konzentration von 10 bis 60 Gew.-% und ganz besonders bevorzugt in einer Konzentration von 10 bis 40 Gew.-%, jeweils bezogen auf das Gesamtgewicht der Zubereitung vor der Trocknung, in der erfindungsgemäßen Emulsion enthalten ist.

Es ist des Weiteren erfindungsgemäß bevorzugt, wenn ein oder mehrere wasserlösliche Polymere in einer Gesamtkonzentration von 1 bis 80 Gew.-%, besonders bevorzugt in einer Konzentration von 2 bis 50 Gew.-%, und ganz besonders bevorzugt in einer Konzentration von 2 bis 25 Gew.-%, jeweils bezogen auf das Gesamtgewicht der Emulsion vor der Trocknung enthalten sind.

Ferner ist es erfindungsgemäß bevorzugt, wenn der Wassergehalt in der Zubereitung nach der Trocknung weniger als 8 Gew.-% und besonders bevorzugt weniger als 5 Gew.-%, jeweils bezogen auf das Gesamtgewicht der getrockneten Emulsion beträgt.

Desweiteren kann es erfindungsgemäß vorteilhaft sein, die getrocknete, pulverförmige Emulsion mit Hilfstoffen, wie zum Beispiel Fließmitteln, Verdickungsmitteln, wasserlöslichen bzw. wasserdispergierbaren Zusätzen, zu vermischen.

Überraschend und für den Fachmann nicht vorhersehbar ist, dass sich die trockenen Emulsionen durch einfachen Zusatz von Wasser "reemulgieren" (verdünnen) lassen, das heißt, dass wieder eine flüssige O/W-Emulsion ausgebildet wird, was für gewöhnlich bei "eingetrockneten" Emulsionen nicht der Fall ist.

Zwar beschreiben Y. Kawashima et al [Int. J. Pharmazeut., 86 (1992) 25-33, Drug Development and Industrial Pharmacy, 18(9), 919-937 (1992) und Chem. Pharm. Bull. 39(6) 1528-1531 (1991)] sowie H.G. Kristensen et al. [Euro. J. Pharmaceutis and Biopharmaceutics 53 (2002) 147-153 und Int. J. Pharmaceutics 212 (2001) 187-194, 195-202] getrocknete, redispergierbare Emulsionen. Auch diese Emulsionen werden mit Hilfe von Hydroxypropylmethylcellulose (wasserlösliches Polymer) stabilisiert. Doch sind die dort beschriebenen Emulsionen bei weitem nicht so komplex aufgebaut wie kosmetische und/oder dermatologische Emulsionen. Daher konnten diese Schriften keinen Weg zur vorliegenden Erfindung weisen.

In der erfindungsgemäßen Zubereitung wird die aus dem Stand der Technik bekannte Koaleszenz der Öltröpfchen durch die synergistische Wirkung von wasserlöslichen und öllöslichen Polymeren verhindert. Das öllösliche Polymer, z.B. Ethylcellulose, stabilisiert das Öltröpchen von innen. Die wasserlöslichen Polymere, z.B. Hydroxypropylmethylcellulose, wirken in der nach der Trocknung verbleibenden Wasserphase als ,Abstandshalter' (Spacer) die den Kontakt der Öltröpfchen untereinander verhindern.

Die erfindungsgemäßen Emulsionen weisen gegenüber den bisher bekannten, redispergierbaren Emulsionen ein besonders angenehmes Hautgefühl auf und lassen sich mit einer Vielzahl kosmetischer Wirk-, Hilfs- und Zusatzstoffen kombinieren. So enthalten die erfindungsgemäßen Emulsionen UV-Lichtschutzfilter, die sich bisher nicht in reemulgierbare Emulsionen einarbeiten ließen.

Die Ölphase der erfindungsgemäßen Emulsion, d.h. die lipophilen organischen Bestandteile, werden vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Caprylic/Capric Triglyceride, Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, Cocoglyceride (z. B. Myritol® 331 von Henkel), C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Corapan®TQ von Haarmann & Reimer*).

Auch beliebige Abmischungen solcher Öl- und Wachskomponenten sind vorteilhaft im Sinne der vorliegenden Erfindung einzusetzen.

Die Lipidphase kann die polaren Ölkomponenten erfindungsgemäß in einer Konzentration von bis zu 80 Gew.-% bezogen auf das Gesamtgewicht der Lipidphase enthalten. Die Gewichtsangabe bezieht sich dabei auf die Zusammensetzung der Zubereitung vor der Trocknung.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene und hydrierte Polyisobutene die bevorzugten Substanzen.

Die unpolaren Ölkomponenten können vorteilhaft in einer Konzentration von bis zu 80 Gew.-% bezogen auf das Gesamtgewicht der Lipidphase in den erfindungsgemäßen Emulsionen enthalten sein. Die Gewichtsangabe bezieht sich dabei auf die Zusammensetzung der Zubereitung vor der Trocknung.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel I auszeichnen 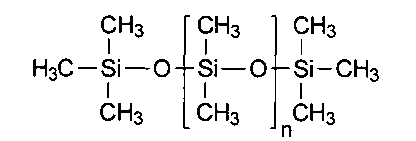 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Erfindungsgemäß besonders bevorzugte Silikone sind Dimethicon und Cyclomethicon.

Der Silikonölanteil der Lipidphase kann vorteilhaft 20 bis 100 Gew.-% und besonders bevorzugt von 30 bis 60 Gew.-%, jeweils bezogen auf das Gesamtgewicht der Lipidphase betragen.

Erfindungsgemäß vorteilhafte wasserlösliche Polymere können erfindungsgemäß vorteilhaft aus der Gruppe der wasserlöslichen bzw. dispergierbaren Filmbildner (z. B. Polyurethane, Dimethicone Copolyol Polyacrylate, Polyvinylpyrrolidon-Vinylacetate PVPNA, Polyvinylpyrrolidone (PVP), Polyvinylalkohole (z.B. Mowiol 18-88) und aus der Gruppe der Hydrokolloide (z.B. Agar-Agar, Carrageen, Tragant, Gummi arabicum, Alginate, Pektine, Polyosen, Guar-Mehl, Johannisbrotbaumkernmehl, Stärke, Dextrine, Polysaccharid-N-alkylurethane, Inulincarbamate, Gelatine, Casein, Celluloseether, Hydroxyethyl- und -propyl-cellulosederivate, Polysaccharide, Polyacryl- und Polymethacryl-Verbindungen, AmmoniumacryloyldimethyltaurateNinylpyrrolidoncopolymere und Ammoniumpolyacryl-dimethyltauramide, Vinylpolymere, Polycarbonsäuren, Polyether, Polyimine, Polyamide, Polykieselsäuren, Tonmineralien, Zeolithe, Kieselsäuren, siehe unten) gewählt werden.

Erfindungsgemäß bevorzugte Hydrokolloide sind beispielsweise Methylcellulosen, als welche die Methylether der Cellulose bezeichnet werden. Sie zeichnen sich durch die folgende Strukturformel II aus 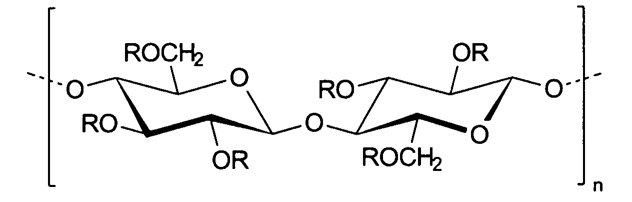 in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugte Hydroxypropylmethylcellulosen (HPMC) weisen eine mittlere Molmasse Mₘ < 50.000 gmol⁻¹ auf und sind beispielsweise unter der Bezeichnung Pharmacoat 603, Pharmacoat 606 und Pharmacoat 645 oder unter der Bezeichnung Metolose 65 SH 50 bzw. Metolose 60 SH 50 bei der FA Shin Etsu erhältlich. Weitere bevorzugte Hydroxypropylmethylcellulosen sind unter dem Handelsnamen Methocel K100LV bei der Firma Dow Chemicals oder unter dem Handelsnamen Methocel E5 bei der Firma Colorcon erhältlich.'

Des weiteren bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das NatriumSalz des Glykolsäureethers der Cellulose, für welches R in Strukturformel II ein Wasserstoff und/oder CH2-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Ferner ist es erfindungsgemäß, wenn eine derartige erfindungsgemäße O/W-Emulsion wasserlösliche kosmetische und/oder dermatologische Wirk-, Hilfs- und/oder Zusatzstoffe enthält.

Erfindungsgemäß vorteilhafte öllösliche Polymere können im Sinne der vorliegenden Erfindung aus der Gruppe der organo-modifizierten Bentonite (z.B. Bentone 38 V von der Firma Nordmann und Rassmann) und/oder aus der Gruppe der hydrophob modifizierten Bentonite (z.B. Tixogel VP-V von der Firma Südchemie) sowie aus der Gruppe der hochdispersen Siliciumdioxiden (Aerosile) gewählt werden. Vorteilhafte Substanzen sind ferner Ethylen/Propylen/Styrol Copolymer, Butylen/Ethylen/Styrol Copolymer (z.B. Versagel® von der Firma Penreco) und/oder Hydriertes Polydecen, Ethylen/Propyle/Styrol Copolymer, Butylen/Ethylen/Styrol Copolymer (z.B. Dekagel® von der Firma Jan Dekker).

Erfindungsgemäß ferner vorteilhaft sind öllösliche, strukturgebende Substanzen wie Allylmethacrylat Crosspolymer (z.B. Poly-Pore L 200® von der Firma Chemdal Corporation), Silikonpolyamid (z.B. Dow Corning 2-8178® von der Firma Dow Corning), Dextrinpalmitat, Polyurethane und Trihydroxystearin .

Erfindungsgemäß besonders bevorzugte öllösliche Polymere können aus der Gruppe der Cellulose-Ether z.B. Ethylcellulose (Kurzzeichen nach DIN 7728-1:1988-01: EC), insbesondere ETHOCEL® der Fa. Dow Chemical Company gewählt werden.

Erfindungsgemäß bevorzugt ist der Einsatz eines oder mehrer öllöslicher Polymere in einer Gesamtkonzentration von 0,1 bis 50 Gew.-%, insbesondere in einer Konzentration von 0,5 bis 10 Gew.-%.

Bei Verwendung einer Kombination von öllöslichen und wasserlöslichen Polymeren hat sich überraschender Weise gezeigt, das sich durch synergistisches Zusammenspiel die Absolutkonzentrationen, der zur Prävention von Phasentrennung notwendigen Inhaltsstoffe, insbesondere der wasserlöslichen und öllöslichen Polymere, herabsetzen lässt.

Ein weiterer Vorteil, der durch die Kombination von öllöslichen und wasserlöslichenm Polymeren erzielt wird, ist der relativ zur Polymerkonzentration höhere Ölanteil in der getrockneten Emulsion und der damit einhergehenden Verbesserung von sensorischen Eigenschaften.

Ein nicht unwesentlicher Nebeneffekt ist, das durch den Zusatz des öllöslichen Polymers, die Wasserfestigkeit, der redispergierten Emulsion auf der Haut, im Vergleich zur redispergierten Emulsion, die lediglich wasserlösliche Polymere enthält, deutlich gesteigert wird.

Erfindungsgemäß ist auch das Verfahren zur Herstellung einer erfindungsgemäßen O/W-Emulsion, welches dadurch gekennzeichnet ist, dass eine durch Walzen-und/oder Kanal- und/oder Sprüh- und/oder Gefriertrocknung getrocknete erfindungsgemäße pulverförmige O/W-Emulsion, in einer Mischungsvorrichtung mit wasserlöslichen und/oder leichflüchtigen Wirk-, Hilfs- und/oder Zusatzstoffen vermischt wird.

Dabei bedeutet "leichtflüchtig" erfindungsgemäß, dass diese Verbindungen einen Siedepunkt von höchstens 100°C aufweisen und somit im Trocknungsprozess azeotrop oder fraktioniert entzogen würden.

Auch eine O/W-Emulsion, die nach einem solchen Verfahren hergestellt wird, ist erfindungsgemäß.

Die erfindungsgemäße Emulsion kann damit erfindungsgemäß wasserlösliche und/oder wasserdispergierbare Inhaltsstoffe enthalten. Es kann erfindungsgemäß vorteilhaft sein diese nach der Trocknung zuzusetzen.

Die erfindungsgemäße Emulsion kann erfindungsgemäß wasserlösliche Inhaltsstoffe enthalten, beispielsweise Alkohole, Diole oder Polyole niedriger C-Zahl, sowie deren Ether, vorzugsweise Ethanol, Isopropanol, Propylenglykol, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Substanzen.

Die Emulsionen können ein oder mehrere Polyole gewählt aus der Gruppe Sorbitol, Mannitol, Propylenglykol sowie Butylenglykol, enthalten.

Die erfindungsgemäß besonders bevorzugten Polyole sind Sorbitol und Mannitol.

Es ist vorteilhaft im Sinne der vorliegenden Erfindung, wenn die erfindungsgemäße kosmetische und/oder dermatologische Emulsion eine Gesamtmenge an Polyolen von 5,0 bis 40,0 Gew.-%, bevorzugt von 7,5 bis 35,0 Gew.-% und ganz besonders bevorzugt von 10,0 bis 25,0 Gew.-%, jeweils bezogen auf das Gesamtgewicht der Zubereitung, enthält. Die Gewichtsangaben beziehen sich dabei auf die Zusammensetzung der Zubereitung vor der Trocknung.

Es ist erfindungsgemäß, dass der Verbraucher der getrockneten Emulsion eine bestimmte Menge Wasser oder eine andere Flüssigkeit zufügt und nach Einwirkung von Bewegungskräften wie z.B. Schütteln oder Rühren eine redispergierte Emulsion erhält.

Die Temperatur des zugefügten Wassers bzw. der anderen Flüssigkeit liegt erfindungsgemäß zwischen 20 und 110 °C.

Die erfindungsgemäße redispergierte Emulsion wird vorteilhaft als Sonnenschutzmittel eingesetzt.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Es ist ebenfalls erfindungsgemäß, dass der Verbraucher das pulverförmige Emulsionskonzentrat als "Körperpuder" direkt, ohne Redispergierung, anwendet.

Dementsprechend enthalten die Zubereitungen im Sinne der vorliegenden Erfindung vorzugsweise mindestens eine UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden, sowie das Sulfat des Bariums (Ba-SO₄).

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegender Erfindung auch in Form kommerziell erhältlicher öliger oder wässriger Vordispersionen zur Anwendung kommen. Diesen Vordispersionen können vorteilhaft Dispergierhilfmittel und/oder Solubilisationsvermittler zugesetzt sein.

Die erfindungsgemäßen Titandioxide zeichnen sich durch eine Primärpartikelgröße zwischen 10 nm bis 150 nm aus und sind unter folgenden Handelsbezeichnungen bei den aufgeführten Firmen erhältlich:

| **Handelsname** | **Coating** | **zusätzliche Bestandteile der Vordispersion** | **Hersteller** |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KgaA |
| Eosolex TS | Alumina, Stearinsäure | - | Merck KgaA |
| Titandioxid P25 | None | - | Degussa |
| Titandioxid T805 (Uvinul TiO₂) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |

Im Sinne der vorliegenden Erfindung sind besonders bevorzugte Titandioxide das MT-100 Z und MT-100 TV von Tayca Corporation, Eusolex T-2000 und Eusolex TS von Merck und das Titandioxid T 805 von Degussa.

Zinkoxide können im Sinne der vorliegenden Erfindung auch in Form kommerziell erhältlicher öliger oder wässriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Die Gesamtmenge an einem oder mehreren anorganischen Pigmenten in der fertigen kosmetischen Zubereitung wird vorteilhaft aus dem Bereich 0,1 Gew.-% bis 25 Gew.-% gewählt, vorzugsweise 0,5 Gew.-% bis 18 Gew.-% bezogen auf die Zubereitung vor der Trocknung.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendi-methylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornyliden-methyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner Benzoxazol-Derivate, welche sich durch die folgende Strukturformel III auszeichnen, 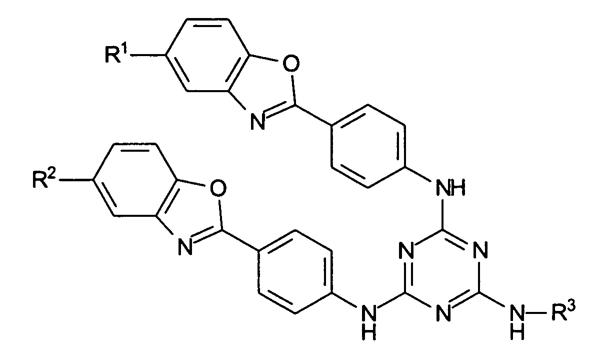 worin R¹, R² und R³ unabhängig voneinander gewählt werden aus der Gruppe der verzweigten oder unverzweigten, gesättigten oder ungesättigten Alkylreste mit 1 bis 10 Kohlenstoffatomen. Es ist erfindungsgemäß besonders vorteilhaft, die Reste R¹ und R² gleich zu wählen, insbesondere aus der Gruppe der verzweigten Alkylreste mit 3 bis 5 Kohlenstoffatomen. Es ist ferner besonders vorteilhaft im Sinne der vorliegenden Erfindung, wenn R³ einen unverzweigten oder verzweigten Alkylrest mit 8 Kohlenstoffatomen, insbesondere den 2-Ethylhexylrest darstellt.

Erfindungsgemäß besonders bevorzugtes Benzoxazol-Derivat ist das 2,4-bis-[5-1 (dimethylpropyl)benzoxazol-2-yl-(4-phenyl)-imino]-6-(2-ethylhexyl)-imino-1,3,5-triazin mit der CAS Nr. 288254-16-0, welches sich durch die Strukturformel IV 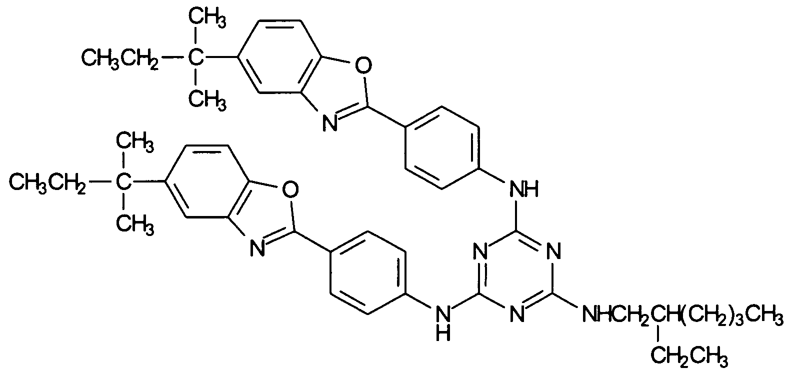 auszeichnet und bei 3V Sigma unter der Handelsbezeichnung Uvasorb® K2A erhältlich ist.

Das oder die Benzoxazol-Derivate liegen vorteilhaft in gelöster Form in den erfindungsgemäßen kosmetischen Zubereitungen vor. Es kann ggf. aber auch von Vorteil sein, wenn das oder die Benzoxazol-Derivate in pigmentärer, d. h. ungelöster Form - beispielsweise in Partikelgrößen von 10 nm bis zu 300 nm - vorliegen.

Vorteilhafte UV-Filtersubstanzen im Sinne der vorliegenden Erfindung sind ferner so genannte Hydroxybenzophenone. Hydroxybenzophenone zeichnen sich durch die folgende Strukturformel V aus: 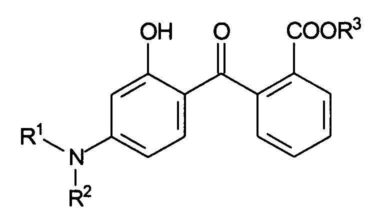 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4-Diethylamino-2-hydoxybenzoyl)-benzoesäurehexylester (auch: Aminobenzophenon), welcher sich durch folgende Struktur VI auszeichnet: 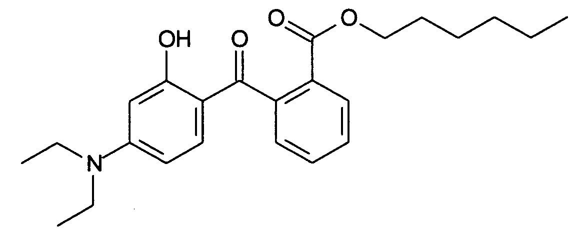 und unter dem Uvinul A Plus bei der Fa. BASF erhältlich ist.

Vorteilhafte UV-Filtersubstanzen im Sinne der vorliegenden Erfindung sind ferner so genannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCI: Diethylhexyl Butamido Triazone), welches unter der Handelsbezeichnung UVASORB HEB bei Sigma 3V erhältlich ist;
- 4,4',4' -(1,3,5-Triazin-2,4,6-triyltriimino)-tris-benzoësäure-tris(2-ethylhexylester), auch: 2,4,6-Tris-[anilino-(p-carbo-2-ethyl-1 -hexyloxy)]-1,3,5-triazin (INCI: Ethylhexyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z.B. unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]di-siloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2-ethyl-1 -hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4 -methylbenzophenon, 2,2 -Dihydroxy-4-methoxybenzophenon sowie
- an Polymere gebundene UV-Filter.

Vorteilhafte wasserlösliche Filtersubstanzen sind z. B.:
Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat, welches von BASF unter der Bezeichnung Uvinul® N 539 T erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A-und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin und/oder Phenylen-1,4-bis-(2-benzimidazyl)-3,3'-5,5'-tetrasulfonsäure-bis-natriumsalz jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 40 Gew.-%, vorzugsweise 0,5 bis 30 Gew.-%, insbesondere 1,0 bis 20 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die Gewichtsangaben beziehen sich dabei auf die Zusammensetzung der Zubereitung vor der Trocknung.

Besonders bevorzugte Ausführungsformen der vorliegenden Erfindung enthalten als UV-Filter ein oder mehrere Triazin-Derivate, Dibenzoylmethanderivate, bei Raumtemperatur flüssige UV-Filter und/oder anorganische Pigmente, insbesondere Titandioxid.

Des Weiteren ist es erfindungsgemäß, dass der Anwender die getrocknete Emulsion ohne weiteren Wasserzusatz direkt z.B. als "Körper-Puder" anwendet.

Ferner kann es gegebenenfalls von Vorteil sein, Filmbildner in die erfindungsgemäße Emulsion einzuarbeiten, beispielsweise um die Wasserfestigkeit der Zubereitungen zu verbessern oder die UV-Schutzleistung zu erhöhen (UV-A- und/oder UV-B-Boosting). Geeignet sind sowohl wasserlösliche bzw. dispergierbare als auch fettlösliche Filmbildner, jeweils einzeln oder in Kombination miteinander.

Erfindungsgemäß vorteilhafte wasserlöslich bzw. dispergierbare Filmbildner, welche auch als erfindungsgemäße wasserlösliche Polymere eingesetzt werden können, sind z. B. Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF), Polyvinylalkohole etc.

Vorteilhafte wasserlösliche Filmbildner sind z. B., die Filmbildner aus der Gruppe der Polymere auf Basis von Polyvinylpyrrolidon (PVP), mit der Struktur VII:

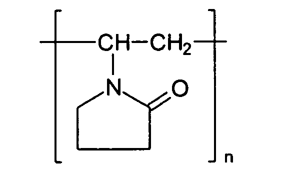

Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Erfindungsgemäß können in der Emulsion die üblichen Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,025 bis 6.0 Gew.-%, insbesondere 0.05 bis 3.0 Gew.-%, bezogen auf das Gesamtgewicht der Zubereitung. Die Gewichtsangaben beziehen sich dabei auf die Zusammensetzung der Zubereitung vor der Trocknung.

Sofern Vitamin A bzw. Vitamin-A-Derivate, bzw. Carotine bzw. deren Derivate das oder die Antioxidantien darstellen, ist es vorteilhaft, deren jeweilige Konzentrationen aus dem Bereich von 0,001 bis 30 Gew.-%, bezogen auf das Gesamtgewicht der Formulierung, zu wählen. Die Gewichtsangaben beziehen sich dabei auf die Zusammensetzung der Zubereitung vor der Trocknung.

Sofern Vitamin E und/oder dessen Derivate das oder die Antioxidantien darstellen, ist es vorteilhaft, deren jeweilige Konzentrationen aus dem Bereich von 0,001 bis 30 Gew.-%, bezogen auf das Gesamtgewicht der Formulierung, zu wählen. Die Gewichtsangaben beziehen sich dabei auf die Zusammensetzung der Zubereitung vor der Trocknung.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Kreatinin, Vitamin C, Licocalcon, Carnosin, natürliche und/oder synthetische Isoflavonoide, Kreatin, Fumarsäureester, Ectoin und dessen Derivate, Taurin, und/oder β-Alanin. Diese Wirkstoffe können in einer Konzentration von 0,001 bis 30 Gew.-%, bezogen auf das Gesamtgewicht der Emulsion, in dieser enthalten sein. Die Gewichtsangaben beziehen sich dabei auf die Zusammensetzung der Zubereitung vor der Trocknung.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

In die erfindungsgemäßen Zubereitungen können aber auch andere pharmazeutisch oder dermatologisch wirkende Substanzen wie beispielsweise die Haut beruhigende und pflegende Substanzen eingearbeitet sein. Hierzu zählen beispielsweise Panthenol, Allantoin, Tannin, Antihistaminika (z.B. Loratadin, Cetirizin, Dimetionden, Clemastin, Capsaicin, H₁-Antagonisten, Gerbstoffpräparate), Lokalanästhetika, Opiatantagonisten (z.B. Naltrexon, Naloxon), Antiphlogistika, Glucocorticoide (z.B. Hydrocortison, Tacrolimus, Ciclosporin A) sowie Pflanzenwirkstoffe wie Azulen und Bisabolol, Glycyrrhizin, Hamamelin und Pflanzenextrakte wie Kamille, aloe vera, Hamazelis, Süßholzwurzel (Glycyrhiza glabra und inflata). Auch die Vitamin D₃-analoga Tacalcitol, Calcipotriol, Tacalcitol, Colecalciferol sowie Calcitrol (Vitamin D₃) und/oder Fumarsäureester können erfolgreich in die Zubereitungen eingearbeitet werden.

Diese Wirkstoffe können in einer Konzentration von 0,001 bis 30 Gew.-%, bezogen auf das Gesamtgewicht der Emulsion, in dieser enthalten sein. Die Gewichtsangaben beziehen sich dabei auf die Zusammensetzung der Zubereitung vor der Trocknung.

Vorteilhafte anfeuchtende bzw. feuchthaltende Mittel (sogenannte Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Vorteilhaft können die erfindungsgemäßen Zubereitungen Feststoffträger in Form von mikrofeinen Feststoffteilchen enthalten. Diese können erfindungsgemäß vorteilhaft oberflächlich wasserabweisend behandelt ("gecoatet") sein, wobei ein amphiphiler Charakter dieser Feststoffteilchen gebildet werden bzw. erhalten bleiben soll. Die Oberflächenbehandlung kann darin bestehen, dass die Feststoffteilchen nach an sich bekannten Verfahren mit einer dünnen hydrophoben bzw. hydrophilen Schicht versehen werden.

Der mittlere Partikeldurchmesser der als Stabilisator verwendeten mikrofeinen Feststoffträger wird vorzugsweise kleiner als 100 µm, besonders vorteilhaft kleiner als 50 µm gewählt. Dabei ist es im wesentlichen unerheblich, in welcher Form (Plättchen, Stäbchen, Kügelchen etc.) bzw. Modifikation die verwendeten Feststoffteilchen vorliegen.

Vorzugsweise werden die mikrofeinen Feststoffträger aus der Gruppe der amphiphilen Metalloxidpigmente gewählt. Vorteilhaft sind insbesondere:
- Titandioxide (gecoatet und ungecoatet): z. B. Eusolex T-2000 von der Fa. Merck, Titandioxid MT-100 Z von der Fa. Tayca Corporation
- Zinkoxide z. B. Z-Cote und Z-Cote HP1 von der BASF AG, MZ -300, MZ -500 und MZ-505M von der Fa. Tayca Corporation
- Eisenoxide
Des weiteren ist es vorteilhaft, wenn die mikrofeinen Feststoffträger aus der folgenden Gruppe gewählt werden: Bornitride, Stärkederivate (Tapioca Starch, Sodium Corn Starch Octynylsuccinat etc.), Talkum, Latexpartikel.

Die mikrofeinen Feststoffteilchen werden erfindungsgemäß vorteilhaft in einer Konzentration von 0,5 bis 60 Gew.-%, bevorzugt in einer Konzentration von 1 bis 50 Gew.-% und besonders bevorzugt in einer Konzentration von 3 bis 30 Gew.-%, jeweils bezogen auf das Gesamtgewicht der Zubereitung eingesetzt. Die Gewichtsangaben beziehen sich dabei auf die Zusammensetzung der Zubereitung vor der Trocknung.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Repellentien, Selbstbräuner (z.B. DHA), Depigmentiermittel (z.B. 8-Hexadecen-1,16-dicarbonsäure (Dioic acid, CAS-Nummer 20701-68-2; vorläufige INCI-Bezeichnung Octadecendioic acid)), Pigmente, die eine färbende Wirkung haben, weichmachende, anfeuchtende und/oder feuchthaltende Substanzen, oder andere übliche Bestandteile einer kosmetischen oder dermatologischen Formulierung wie Emulgatoren, Polymere, Schaumstabilisatoren, Peeling-Stoffe (Abrasiva, z.B. Polymerkügelchen oder -pulver aus Polyethylen, Polypropylen etc. anorganischen Oxiden, Silikaten usw.), Antitranspirant-Salze (z.B. saure Aluminium- und/oder Aluminium/Zirkoniumsalze wie Aluminiumchlorhydrat und/oder Aluminium/Zirkoniumchlorhydrat) und Elektrolyte.

Erfindungsgemäß bevorzugt sind die im folgenden aufgelisteten Repellent-Wirkstoffe:

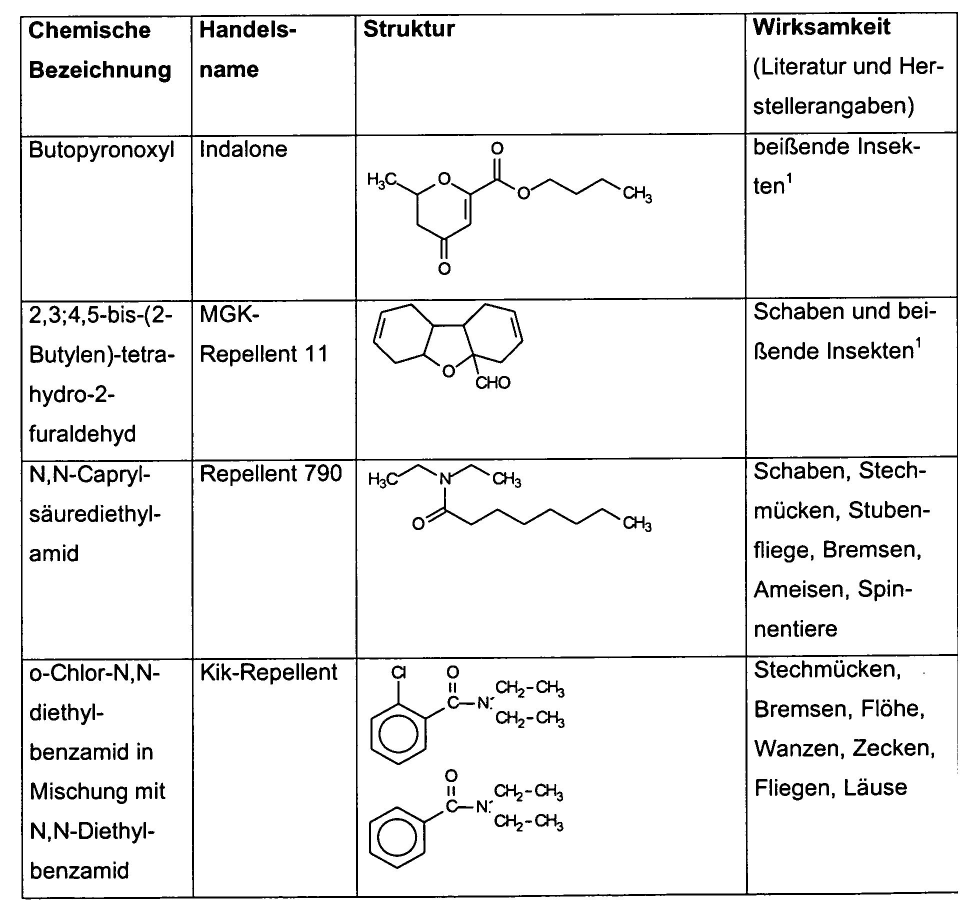

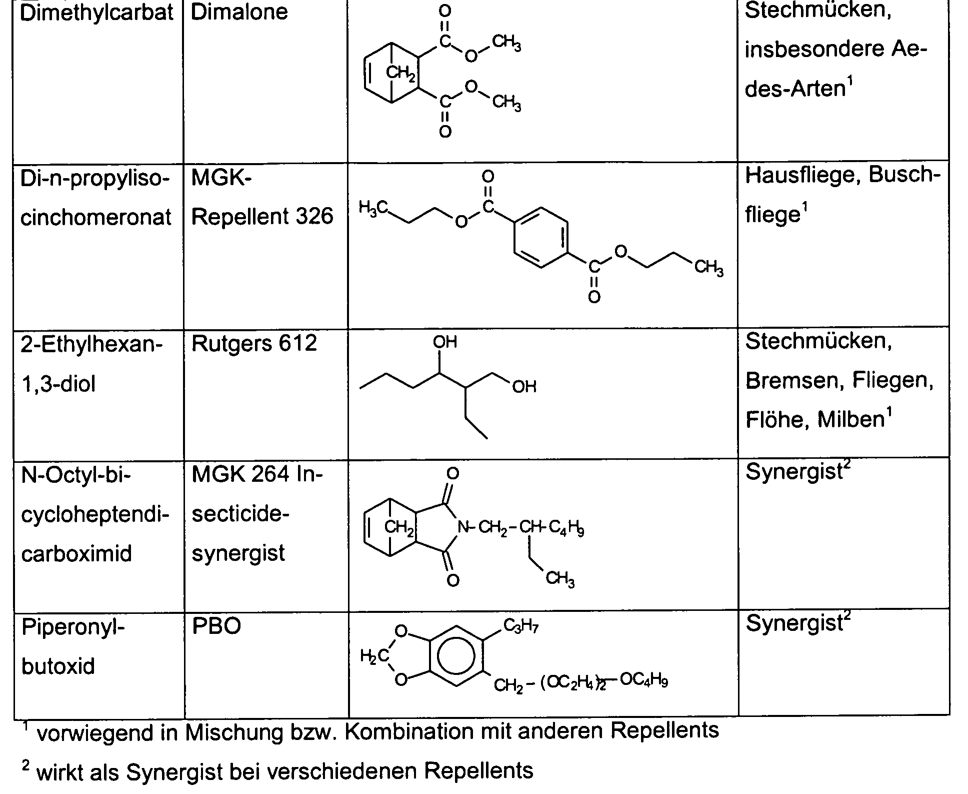

Besonders vorteilhafte Repellent-Wirkstoffe im Sinne der vorliegenden Erfindung sind die obengenannten Wirkstoffe N,N-Diethyl-3-methylbenzamid, 3-(N-n-Butyl-N-acetyl-amino)propionsäureethylester und Dimethylphthalat. Ganz besonders bevorzugt ist das Repellent 3-(N-n-Butyl-N-acetyl-amino)propionsäureethylester.

Erfindungsgemäß vorteilhafte Ausführungsformen der erfindungsgemäßen Emulsion enthalten ein oder mehrere Repellent-Wirkstoffe in einer Konzentration von 1 bis 50 Gew.-%, bezogen auf das Gesamtgewicht der Formulierung. Die Gewichtsangaben beziehen sich dabei auf die Zusammensetzung der Zubereitung vor der Trocknung.

Als Selbstbräuner können erfindungsgemäß vorteilhaft unter anderem eingesetzt werden:

Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon), 2-Hydroxy-1,4-naphtochinon (Lawson) und besonders bevorzugt 1,3-Dihydroxyaceton.

Erfindungsgemäß vorteilhafte Ausführungsformen mit mindestens einer Selbstbräunungssubstanz, enthalten diese in einer Gesamtkonzentration von 0,1 bis 30 Gew.-%, bezogen auf das Gesamtgewicht der Emulsion. Die Gewichtsangaben beziehen sich dabei auf die Zusammensetzung der Zubereitung vor der Trocknung.

Die erfindungsgemäße Emulsion kann erfindungsgemäß vorteilhaft ein oder mehrere Konservierungsstoffe enthalten. Vorteilhafte Konservierungsstoffe im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Die nachfolgende Tabelle gibt einen Überblick über einige erfindungsgemäß vorteilhafte Konservierungsstoffe:

| | | | |
|---|---|---|---|
| E 200 | Sorbinsäure | E 227 | Kalziumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Kalziumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Kalziumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Kalziumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Kalziumpropionat |
| E 224 | Kaliumdisulfit | E 283 | Kaliumpropionat |
| E 226 | Kalziumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, Salicylsäure und Salicylate.

Es ist dabei erfindungsgemäß besonders bevorzugt, wenn als Konservierungsstoffe lodopropylbutylcarbamate, Parabene (Methyl-, Ethyl-, Propyl- und/oder Butylparaben) und/oder Phenoxyethanol eingesetzt werden.

Erfindungsgemäß vorteilhaft sind ein oder mehrere Konservierungsstoffe in einer Konzentration von 0,001 bis 2 Gew.-%, bevorzugt 0,01 bis 1,5 Gew.-% und besonders bevorzugt 0,05 bis 1 Gew.-%, jeweils bezogen auf das Gesamtgewicht der Zubereitung enthalten. Die Gewichtsangaben beziehen sich dabei auf die Zusammensetzung der Zubereitung vor der Trocknung.

Die erfindungsgemäße Emulsion enthält vorteilhafter Weise einen oder mehrere Konditionierer. Erfindungsgemäß bevorzugte Konditionierer sind beispielsweise alle Verbindungen, welche im *International Cosmetic Ingredient Dictionary and Handbook* (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen. Ein Teil dieser Verbindungen wird unter den Bestandteilen der wässrigen Phase und der Ölphase namentlich aufgeführt. Weitere erfindungsgemäß vorteilhafte Konditionierer stellen beispielsweise die nach der internationalen Nomenklatur für kosmetische Inhaltsstoffe (INCI) als Polyquaternium benannten Verbindungen dar (insbesondere Polyquaternium-1 bis Polyquaternium-56).

Erfindungsgemäß vorteilhaft kann die erfindungsgemäße Emulsion Glittersoffe und/oder andere Effektstoffe enthalten.

Als erfindungsgemäß vorteilhafte Hydrokolloide, die als erfindungsgemäße wasserlösliche Polymere eingesetzt werden können, werden Agar-Agar, Carrageen, Tragant, Gummi arabicum, Alginate, Pektine, Polyosen, Guar-Mehl, Johannisbrotbaumkernmehl, Stärke, Dextrine, Polysaccharid-N-alkylurethane, Inulincarbamate, Gelatine, Casein, Celluloseether, Hydroxyethyl- und -propyl-cellulosederivate, Polysaccharide, Polyacryl- und Polymethacryl-Verbindungen, Ammoniumacryloyldimethyltaurate/Vinylpyrrolidoncopolymere und Ammoniumpolyacryl-dimethyltauramide, Vinylpolymere, Polycarbonsäuren, Polyether, Polyimine, Polyamide, Polykieselsäuren, Tonmineralien, Zeolithe, Kieselsäuren eingesetzt.

Ein vorteilhaftes Hydrokolloid im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird.

Ein vorteilhaftes Hydrokolloid im Sinne der vorliegenden Erfindung ist ferner Carrageen, ein gelbildender und ähnlich wie Agar aufgebauter Extrakt aus nordatlant. - zu den Florideen zählenden - Rotalgen (Chondrus crispus u. Gigartina stellata).

Polyacrylate sind ebenfalls vorteilhaft im Sinne der vorliegenden Erfindung zu verwendende Hydrokolloide. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alkylacrylat-Copolymere durch die folgende Struktur VIII aus:

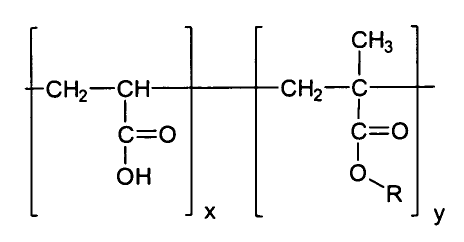

Darin stellen R' einen langkettigen Alkylrest und x und y Zahlen dar, welche den jewieligen stöchiometrischen Anteil der jeweiligen Comonomere symbolisieren.

Erfindungsgemäß bevorzugt sind Acrylat-Copolymere und/oder Acrylat-Alkylacrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind. Weiterhin bevorzugt sind die Carbomere Carbopol® EDT 2001, ETD 2020 und ETD 2050.

Ferner vorteilhaft sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Pemulen® TR1 und Pemulen® TR2 bei der NOVEON Inc. erhältlichen.

Vorteilhaft sind Verbindungen, die die INCI- Acrylates/Vinyl Isodecanoate Crosspolymer" tragen. Insbesondere vorteilhaft sind die unter den Handelsbezeichnungen Stabylen 30 von 3V Sigma erhältlichen.

Vorteilhaft sind ferner Verbindungen, die die INCI-Bezeichnung "acrylates/C12-24 pareth-25 acrylate copolymer" (unter der Handelsbezeichnungen Synthalen® W2000 bei der 3V Inc. erhältlich), die die INCI-Bezeichnung "acrylates/steareth-20 methacrylate copolymer" (unter der Handelsbezeichnungen Aculyn® 22 bei der International Specialty Products Corp. erhältlich), die die INCI-Bezeichnung "acrylates/steareth-20 itaconate copolymer" (unter der Handelsbezeichnungen Structure 2001® bei der National Starch erhältlich), die die INCI-Bezeichnung "acrylates/aminoacrylates/C10-30 alkyl PEG-20 itaconate copolymer" (unter der Handelsbezeichnungen Structure Plus® bei der National Starch erhältlich) und ähnliche Polymere.

Erfindungsgemäß bevorzugt ist es insbesondere, neutralisierte oder teilneutralisierte Polyacrylate (z.B. Carbopole der Firma Noveon) einzusetzen.

Die erfindungsgemäßen kosmetischen und/oder dermatologischen Emulsionen können eine Reihe von Pigmenten enthalten.

Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem *Rowe Colour Index, 3. Auflage, Society of Dyers and Colourists, Bradford, England, 1971* entnommen.

| **Chemische oder sonstige Bezeichnung** | **CIN** | **Farbe** |
|---|---|---|
| Pigment Green | 10006 | Grün |
| Acid Green 1 | 10020 | Grün |
| 2,4-Dinitrohydroxynaphthalin-7-sulfosäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'-sulfosäurediethylamid-1'-phenylazo)-3-hydroxy-5"-chloro-2",4"-dimethoxy-2-naphthoesäureanilid | 12490 | Rot |
| Disperse Yellow 16 | 12700 | Gelb |
| 1-(4-Sulfo-1-phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosäure)-1-hydroxynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | Rot |
| 1-(2-Sulfo-4-methyl-5-chlor-1-phenylazo)-2-hydroxynaphthalin-3-carbonsäure | 15865 | Rot |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2 -phenylazo- 1 -naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxy-pyrazolon-3-carbonsäure | 19140 | Gelb |
| Pigment Yellow 16 | 20040 | Gelb |
| 2,6-(4'-Sulfo-2", 4"-dimethyl)-bis-phenylazo)1,3-dihydroxy-benzol | 20170 | Orange |
| Acid Black 1 | 20470 | Schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1"-phenylazo)-7'-sulfo-1'-naphthylazo]-1-hydroxy-7-aminonaphthalin-3,6-disulfosäure | 27755 | Schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-β-Apo-8'-Carotinaldehyd (C₃₀) | 40820 | Orange |
| trans-Apo-8'-Carotinsäure (C₃₀)-ethylester | 40825 | Orange |
| Canthaxanthin | 40850 | Orange |
| Acid Blue 1 | 42045 | Blau |
| 2,4-Disulfo-5-hydroxy-4'-4"-bis-(diethylamino)triphenyl-carbinol | 42051 | Blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl)Δ^{2,5}-cyclohexadienimin | 42090 | Blau |
| Acid Green 9 | 42100 | Grün |
| Diethyl-di-sulfobenzyl-di-4-amino-2-chlor-di-2-methyl-fuchsonimmonium | 42170 | Grün |
| Basic Violet 14 | 42510 | Violett |
| Basic Violet 2 | 42520 | Violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | Blau |
| 4'-(N-Dimethyl)-amino-4"-(N-phenyl)-aminonaphtho-N-dimethyl-fuchsonimmonium | 44045 | Blau |
| 2-Hydroxy-3,6-disulfo-4,4'-bis-dimethylamino-naphthofuchsonimmonium | 44090 | Grün |
| Acid Red 52 | 45100 | Rot |
| 3-(2'-Methylphenylamino)-6-(2'-methyl-4'-sulfophenylamino)-9-(2"-carboxyphenyl)-xantheniumsalz | 45190 | Violett |
| Acid Red 50 | 45220 | Rot |
| Phenyl-2-oxyfluoron-2-carbonsäure | 45350 | Gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |

Es kann ferner günstig sein, als Farbstoff eine oder mehrer Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4-sulfosäure, Kalziumsalz der 2-Hydroxy-1,2"azonaphthalin-1-'ulfosäure, Kalzium- und Bariumsalze der 1-(2-Sulfo-4-methyl-1-phenylazo)-2-naphthylcarbonsäure, Kalziumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 8-Amino-2 -phenylazo- 1 -naphthol-3,6-disulfosäure , Aluminiumsalz der 4-(4-Sulfo-1-phenylazo)-1-(4-sulfophenyl)-5-hydroxy-pyrazolon-3-carbonsäure, 4'-[(4"-Sulfo-1"-phenylazo)-7'-sulfo-1'-naphthylazo]-1-hydroxy-8-acetylaminonaphthalin-3,5-disulfosäure, Aluminium- und Zirkoniumsalze von 4,5-Dibromfluorescein, Aluminium- und Zirkoniumsalze von 2,4,5,7-Tetrabromfluorescein, 3"4"5"6"Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, 4,4-Di'ethyl-6,6-di'hlorthioindigo, Komplexsalz (Na, Al, Ca) der Karminsäure, rotes und schwarzes Eisenoxid (CIN: 77 491 (rot) und 77 499 (schwarz)), Eisenoxidhydrat (CIN: 77 492), Manganammoniumdiphosphat (CIN 77745), Ultramarin (CIN 77007) und Titandioxid.

Erfindungsgemäße Emulsionen können Titandioxide enthalten, die sowohl in der Kristallmodifikation Rutil als auch Anatas vorliegen können und im Sinne der vorliegenden Erfindung vorteilhaft oberflächlich behandelt ("gecoatet") ist, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Zirkoniumoxid (ZrO₂) oder Eisenoxid (Fe₂O₃). Diese anorganischen Oberflächenbeschichtungen können allein, in Kombination und/oder in Kombination mit organischen Beschichtungsmaterialien vorkommen.

Hierzu werden Oxide, Oxidhydrate oder Phosphate beispielsweise der Elemente Al, Si, Zr in dichten Schichten auf die Pigmentoberfläche aufgefällt.

Die anorganische Nachbehandlung geschieht im allgemeinen in einer wässrigen Suspension des Pigmentes durch Zugabe löslicher Nachbehandlungschemikalien, wie z.B.

Aluminiumsulfat, und anschließende Ausfällung des im neutralen Bereich schwerlöslichen Hydroxides durch gezielte Einstellung des pH-Wertes mit Natronlauge.

Nach der anorganischen Nachbehandlung werden die gecoateten Pigmente durch Filtration aus der Suspension abgetrennt und sorgfältig gewaschen, um die gelösten Salze zu entfernen, anschließend werden die isolierten Pigmente getrocknet.

Besonders bevorzugt im Sinne dieser Erfindung sind Titandioxide, auf die Aluminiumhydroxid auf die Oberfläche aufgebracht worden ist, wie z.B. die von Sun Chemical erhältlichen Titandioxid Typen C47-051 und C47- 5175. Weiterhin bevorzugte Pigmente sind Titandioxide, die mit Aluminium- und / oder Siliziumoxiden gecoated sind, wie z.B. von der Firma Krosnos Titan: Kronos 1071 und 1075 oder von der Firma Kingfisher: A310.03 Tudor Aspen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Ferner kann es erfindungsgemäß vorteilhaft sein Perlglanzpigmente einzusetzen.
Dazu zählen natürliche Perlglanzpigmente, wie z. B.
■ "Fischsilber" (Guanin/Hypoxanthin-Mischkristalle aus Fischschuppen) und
■ "Perlmutt" (vermahlene Muschelschalen),
monokristalline Perlglanzpigmente wie z. B. Bismuthoxychlorid (BiOCl), Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| **Silberweiße Perlglanz- pigmente** | TiO₂: 40 - 60 nm | silber |
| **Interferenzpigmente** | TiO₂: 60 - 80 nm | gelb |
| | TiO₂: 80 - 100 nm | rot |
| | TiO₂: 100 - 140 nm | blau |
| | TiO₂: 120 - 160 nm | grün |
| **Farbglanzpigmente** | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| **Kombinationspigmente** | TiO₂ / Fe₂O₃ | Goldtöne |
| | TiO₂ / Cr₂O₃ | grün |
| | TiO₂ / Berliner Blau | tiefblau |
| | TiO₂ / Carmin | rot |

Besonders bevorzugt sind die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden.

Es kann darüber hinaus von Vorteil sein, gänzlich auf ein Substrat wie Glimmer zu verzichten. Besonders bevorzugt sind Perlglanzpigmente, welche unter der Verwendung von SiO₂ hergestellt werden. Solche Pigmente, die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Kalzium Natrium Borosilikat, die mit Titandioxid beschichtet sind, eingesetzt werden. Diese sind unter dem Namen Reflecks erhältlich. Sie weisen durch ihrer Partikelgröße von 40 - 180 µm zusätzlich zu der Farbe einen Glitzereffekt auf.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen. Die Gewichtsangaben beziehen sich dabei auf die Zusammensetzung der Zubereitung vor der Trocknung.

Vorteilhaft im Sinne der vorliegenden Erfindung kann die erfindungsgemäße O/W-Emulsion einen oder mehrere Tablettenhilfsstoffe enthalten. Diese können erfindungsgemäß vorteilhaft in einer Konzentration von 0,1 bis 60 Gew.-%, bevorzugt in einer Konzentration von 0,1 bis 50 Gew.-% und ganz besonders bevorzugt in einer Konzentration von 0,5 bis 35 Gew.-%, jeweils bezogen auf das Gesamtgewicht der Zubereitung eingesetzt werden. Die Gewichtsangaben beziehen sich dabei auf die Zusammensetzung der Zubereitung wobei als Zubereitung die Emulsion nach der Trocknung zusammen mit evtl. weiteren wasserlöslichen und/oder leichtflüchtigen Verbindungen und Tablettierstoffen (inkl. Hydrogencarbonaten und bei Raumtemperatur festen Säuren) angesehen wird.

Als Tablettenhilfsstoffe können beispielsweise Füll- und Kompaktierhilfsstoffe (z.B. Stärke- und/oder Cellulosederivate), Fließmittel (z.B.hochdisperse Siliziumdioxide), Fließreguliermittel, Schmiermittel, Formtrennmittel eingesetzt werden. Diese Tabletten-hilfsstoffe können ausdrücklich auch dann zugesetzt werden, wenn das Produkt als Pulver belassen und nicht zur Tablette weiterverarbeitet wird. Besonders bevorzugte Fließmittel sind beispielsweise unter der Bezeichnung Sipernat bei der Firma Degussa erhältlich.

Erfindungsgemäß vorteilhaft können alle Stoffe eingesetzt werden, die im H.P. Fiedler, Lexikon der Hilfsstoffe für Pharmazie, Kosmetik und angrenzenden Gebiete, 5. Auflage, Editio Cantor Verlag, Aulendorf, 2002, unter den Stichworten Tabletten-Bindemittel, Tabletten-Füllstoffe, Tabletten-Gleitmittel, Tabletten-Hilfsstoffe, Tabletten-Sprengmittel, Tabletten-Überzüge aufgelistetet sind.

In einer erfindungsgemäß besonders bevorzugten Ausführungsform enthält die erfindungsgemäße Zubereitung eine Kombination aus Carbonaten und/oder Hydrogencarbonaten sowie bei Raumtemperatur festen Säuren (z.B. Zitronensäure, Ascorbinsäure, Milchsäure, Weinsäure, etc). Auch stark quellende Polymere wie beispielsweise vernetztes Polyvinylpyrrolidon kann erfindungsgemäß vorteilhaft eingesetzt werden.

Erfindungsgemäß vorteilhaft werden ein oder mehrere Hydrogencarbonate (z.B. Natriumhydrogencarbonat, Kaliumhydrogencarbonat) in einer Menge von 0,1 bis 30 Gew.-%, bevorzugt in einer Menge von 0,5 bis 20 Gew.-% und besonders bevorzugt in einer Menge von 0,5 bis 15 Gew.-%, jeweils bezogen auf das Gesamtgewicht der Zubereitung eingesetzt, wobei als Zubereitung die Emulsion nach der Trocknung zusammen mit evl. weiteren wasserlöslichen und/oder und/oder leichtflüchtigen Verbindungen und Tablettierstoffen (inkl. Hydrogencarbonaten und bei Raumtemperatur festen Säuren) angesehen wird.

Erfindungsgemäß vorteilhaft werden ein oder mehrere bei Raumtemperatur feste Säuren (besonders bevorzugt Zitronensäure) in einer Menge von 0,1 bis 30 Gew.-%, bevorzugt in einer Menge von 0,5 bis 20 Gew.-% und besonders bevorzugt in einer Menge von 0,5 bis 15 Gew.-%, jeweils bezogen auf das Gesamtgewicht der Zubereitung eingesetzt, wobei als Zubereitung die Emulsion nach der Trocknung zusammen mit evtl. weiteren wasserlöslichen und/oder leichtflüchtigen Verbindungen und Tablettierstoffen (inkl. Hydrogencarbonaten und bei Raumtemperatur festen Säuren) angesehen wird.

Eine derartige Ausführungsform ist insbesondere dann vorteilhaft im Sinne der vorliegenden Erfindung, wenn die erfindungsgemäße Emulsion in Form einer Tablette (z.B. einer Brausetablette) vorliegt, da sich die erfindungsgemäße Emulsion in dieser Ausführungsform besonders leicht durch Zusatz von Wasser reemulgieren lässt.

Erfindungsgemäß ist auch das Verfahren zur Herstellung einer derartigen Zubereitung, welches dadurch gekennzeichnet ist, dass die erfindungsgemäße O/W-Emulsion mit einem oder mehreren Tablettenhilfsstoffen nach dem Baukastenprinzip zusammengesetzt und in einer Mischungsvorrichtung vermischt werden. Dieser Prozess des Vermischens der getrockneten Emulsion mit Hilfsstoffen, wird im Sinne der Erfindung als Abmischung zum verkehrsfähigen Produkt verstanden. Die Abmischung hat zum Zweck ein stabiles, je nach Anwendungsbereich rieselfähiges, in Tabletten pressbares und/oder lagerfähiges Produkt zu erhalten. Insbesondere der Zusatz, also die Abmischung, von leichtflüchtigen Substanzen, wie z.B. Parfümstoffen, ist erst nach der Trocknung möglich. Auch ist es zuweilen nötig Trennmittel, die die Rieselfähigkeit erhalten zuzusetzen.

Insbesondere bei der Sprühtrocknung entstehen voluminöse Produkte, deren Schüttdichte durch Zusatz von Tablettierhilfsstoffen erhöht werden kann.

Erfindungsgemäß sind auch Zubereitungen, die nach diesem erfindungsgemäßen Verfahren hergestellt werden.

Erfindungsgemäß ist ferner ein Verfahren zur Herstellung von Tabletten und/oder Granulaten, welches dadurch gekennzeichnet ist, dass eine erfindungsgemäße Zubereitung in einer Presse zu einer oder mehreren Tabletten gepresst oder granuliert wird, sowie Zubereitungen, die nach diesem Verfahren hergestellt werden.

Erfindungsgemäß ist nicht zuletzt das Verfahren zur Herstellung eines Kosmetikums, welches dadurch gekennzeichnet ist, dass eine erfindungsgemäße Zubereitung (eine getrocknete O/W-Emulsion, die gegebenenfalls mit wasserlöslichen und/oder leichtflüchtigen Wirk-, Hilfs- Zusatzstoffen und/oder Tablettenhilfsstoffen versetzt sowie gegebenenfalls tablettiert bzw. granuliert wurde) mit Wasser versetzt bzw. in Wasser emulgiert wird. Erfindungsgemäß sind auch Kosmetika, die nach diesem Verfahren hergestellt werden.

Bei der Sprühtrocknung wird das zu trocknende Material (flüssige Lsg., Suspension oder Emulsion) wird am oberen Ende eines weiten, zylindrischen Behälters durch Düsen od. mittels einer schnell rotierenden Zerstäuberscheibe zu einem feinen Nebel versprüht. Dem entstehenden Sprühkegel wird heiße Luft od. ein Inertgas (bei Oxidationsempfindlichen Stoffen) von unten entgegengeführt. Bei sehr Temperaturempfindlichen Produkten (z. B. Enzyme, aktive Mikroorganismen) empfiehlt sich die Zufuhr des Trocknungsgases im Gleichstrom von oben. Die große, relative Oberfläche der Flüssigkeitströpfchen bewirkt einen effektiven u. schnellen Wärme- u. Stoffaustausch zwischen den beiden Phasen. Das Trockengut fällt als mehr od. weniger feines Pulver, als Granulat od. in Form kleiner Perlen (sog. Prills) nach unten u. wird am Boden des Trockners durch mechanische Räumer, Transportschnecken od. dgl. ausgetragen. In der Abluft verbliebene Staubpartikel müssen durch Fliehkraft-Abscheider (Zyklone) gewonnen werden. Die Leistung industrieller Sprühtrockner kann 100 t/h Wasserdampf erreichen.

Ziel einer verfahrenstechnischen Optimierung der Sprühtrocknung ist es, die Zufuhrrate von Gas u. Wärme so zu gestalten, dass sie der Verdampfungsrate des Wassers aus den Tröpfchen entspricht.

Unter der Gefriertrocknung (Lyophilisation) versteht man eine Grundoperation der Aufarbeitungstechnik, bei der die flüssige Phase (in den meisten Fällen Wasser) durch Sublimation aus dem gefrorenen Gut unter Umgehung des flüssigen Aggregatzustandes entzogen wird.

Aus dem Phasendiagramm des Wassers ist ersichtlich, dass Sublimation nur unterhalb des Tripelpunktes erfolgen kann. Für die Gefriertrocknung ist daher die Temperatur wesentlich, bei der nur noch eine feste Phase vorliegt (eutektischer Bereich). Bei komplex zusammengesetzten Produkten liegt dieser Punkt meist viel tiefer, als bei Wasser u. der Wasserdampfpartialdruck ist an der Sublimationsfläche sehr gering. Daher ist ein entsprechendes Vakuum für die Gefriertrocknung erforderlich. Während des Trocknungsvorganges darf die Temperatur des zu trocknenden Gutes diesen Wert nicht übersteigen, um ein Auftauen zu verhindern.

Die für die Sublimation benötigte Energie (2845 kJ/kg Wasser) muss während des Trocknungsvorgangs von außen an die Sublimationszone herangeführt werden. Dies geschieht durch *Kontakt,* durch *Strahlung* u., in Abhängigkeit vom Vakuum in der Trockenkammer, durch *Konvektion*. Da unterhalb von 10⁻² mbar praktisch keine Wärmeübertragung mehr durch Konvektion möglich ist, muss das Vakuum stets so schlecht wie möglich gehalten werden, aber noch gut genug, um ein Auftauen zu verhindern. Die Druckregulierung muss daher bei der Gefriertrocknung sehr genau erfolgen.

Die Gefriertrocknung ist sicher die schonendste Trocknung u. wird bei bes. Temperaturempfindlichen Bioprodukten angewendet. Überwiegend erfolgt die Gefriertrocknung in Vakuumtunnel- od. Vakuumteller-Trocknern innerhalb von 1 bis 3 Stunden. Die Vorteile der Gefriertrocknung liegen in den niedrigen Temperatur, bei denen keine chemische Veränderung des Produktes auftritt. Der technische Aufwand (Erzeugung des Vakuums und der tiefen Temperatur von -45 °C im Kondensator) u. der spezifische Energiebedarf sind jedoch hoch, so dass die Anwendung bisher auf kostenintensive Produkte, wie Hormon-, Enzym- u. Vitaminpräp., Plasma- u. Serumkonserven zur Bluttransfusion, Viren, Bakterien (Lebendimpfstoffe, Stammhaltung), angewendet wurde.

Bei der Kanaltrocknung passiert das ausgebreitete Nassgut, mechanisch fortbewegt (Transportband), einen Kanal, der durch Dampf, Heißwasser oder Heißluft beheizt ist. Ventilatoren sorgen für eine Luftumwälzung. Das Trockengut wird am Kanalende entnommen. Auch Trockentrommeln und solche Einrichtungen, bei denen das Trocknungsmaterial durch Rührer oder andere Einbauten bewegt wird, z.B. Schnecken-, Schaufel- oder Muldentrockner sind vorteilhaft. Sie werden gleichfalls beheizt und gestatten einen kontinuierlichen Materialfluss.

Walzentrockner bewähren sich zum Entfernen der Flüssigkeit aus viskosem Material. Aus einem Vorratsbehältnis wird die Lösung mit Hilfe einer Auftragswalze in dünner Schicht auf eine von innen beheizte Metallwalze aufgebracht und es erfolgt eine Kontakttrocknung. Der Trocknungsprozess dauert nur einige Sekunden, denn nach einer knappen Umdrehung wird das getrocknete Material mit Schabern von der Walze abgenommen. Substanzen, die selbst dieser kurzfristigen Erhitzung nicht standhalten, lassen sich auf Vakuumwalzentrocknern entwässern.

Ein derartiges erfindungsgemäßes Kosmetikum liegt dann nach Trocknung und anschließender erneuter Rehydration in Form einer Salbe, Creme, Lotion oder eines E-mulsionsschaumes (franz. Mousse) oder einer sprühbaren Form vor. Es kann erfindungsgemäß vorteilhaft zur Behandlung und Pflege der Haut, Haare und Nägel eingesetzt werden. Erfindungsgemäß bevorzugt ist dabei die Verwendung als Sonnenschutzmittel.

Es ist auch erfindungsgemäß, die getrocknete Emulsion ohne weiteren Wasserzusatz, also ohne Redispergierung, direkt auf die Haut aufzutragen. Dies kann in Form von z.B. "Körper-Puder" oder Stiften, insbesondere Puder- und/oder Abdeck-Stiften, geschehen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die Beispielzubereitungen 1 bis 5 (Emulsions- Zubereitungen vor Trocknung) können erfindungsgemäß mit Hilfsstoffabmischungen 1 bis 5 (enthaltend wasserlösliche/dipergierbare Zusätze und/oder Tablettier- und Granulierhilfstoffe) nach dem "Baukasten-Prinzip" homogen vermischt werden. Das Mischungsverhältnis von getrockneter Emulsion und Hilfsstoffabmischung wird vorteilhaft im Bereich 1000:1 bis 1:2 gewählt.

### Beispiele:

### Zubereitung 1: Lichtschutzzubereitung

| | **Gew.-%** |
|---|---|
| Ethylcellulose | 0,7 |
| Hydroxypropylmethylcellulose | 25 |
| Titandioxid (Eusolex T2000) | 6 |
| Nylon-12 | 1 |
| Hydrogenierte Coco Glyceride | 2 |
| Caprylic/Capric Triglycerid | 10 |
| Octyldodecanol | 10 |
| Mineralöl | 3 |
| Butylen Glycol Caprylat/Caprat | 7 |
| C₁₂₋₁₅ Alkyl Benzoat | 6 |
| Cyclomethicon | 2 |
| Starch Hydroxypropyltrimonium Chlorid (Sensomer CI 50) | 1 |
| Bis-Ethylhexyloxyphenol Methoxyphenyl Triazin | 4 |
| Ethylhexyl Methoxycinnamat | 8 |
| Octocrylen | 6 |
| Konservierung | 2 |
| Glycerin | 5 |
| wasserlöslicher Farbstoff | 0,3 |
| Wasser | ad 100 |

### Zubereitung 2: O/W-Selbstbräuner-Emulsion

| **Zubereitung** | **Gew.-%** |
|---|---|
| Ethylcellulose | 0,5 |
| Ethylen/Propylen/Styrol/Copolymer | 0,5 |
| Hydroxypropylmethylcellulose | 30 |
| Xanthan Gummi | 2 |
| C₁₀₋₃₀ Alkyl Acrylat Crosspolymer (Pemulen TR1) | 1 |
| Bornitrid | 2 |
| Di Stärkephosphat | 1 |
| Polyamid-6 | 1 |
| Hydrogenierte Coco Glyceride | 2 |
| Cetyl Dimethicon (Abil Wax 9840) | 2 |
| Caprylic/Capric Triglycerid | 10 |
| Octyldodecanol | 10 |
| Dicaprylyl Carbonate | 5 |
| Stearyl Alcohol | 2 |
| Dimethicon | 2 |
| PPG-15 Stearyl Ether | 1 |
| Hydriertes Polyisobuten (Polysynlan) | 1 |
| Dihydroxyaceton | 4 |
| fettlöslicher Farbstoff | 1 |
| Di-Stärkephosphat | 2 |
| Glycerin | 7 |
| Panthenol | 3 |
| Sorbitol | 5 |
| Milchsäure | 1 |
| Hydroxyethylcellulose | 1 |
| Polyacrylat | 2 |
| Wasser | ad 100 |

### Zubereitung 3: Lichtschutzzubereitung

| | **Gew.-%** |
|---|---|
| Ethylcellulose | 2 |
| Quaternium-90 Bentonit | 0,5 |
| Allyl Methacrylate Crosspolymer | 0,5 |
| Hydroxypropylmethylcellulose | 15 |
| Titandioxid (Eusolex T2000) | 9 |
| Titandioxid (Titandioxid T805) | 5 |
| Behenoxy Dimethicon (Abil Wax 2440) | 2 |
| Caprylic/Capric Triglycerid | 10 |
| Mineralöl | 9 |
| Butylen Glycol Caprylat/Caprat | 9 |
| Dicaprylyl Ether (Cetiol OE) | 3,5 |
| UVASorb® K2A | 3 |
| Ethylhexyl Triazon | 6 |
| Diethylhexyl Butamido Triazon | 5 |
| Phenyl Dibenzimidazol Tetrasulfonsäure | 3 |
| Phenylbenzimidazol Sulfonsäure | 4 |
| Mannitol | 3 |
| Biosaccharid Gel (Fucogel 1000) | 2 |
| Milchsäure | 0,5 |
| Polyquaternium 37 | 2 |
| NaOH 45%ige Lösung in Wasser | 3 |
| Parfum | 1 |
| Wasser | ad 100 |

### Zubereitung 4: Lichtschutzspray

| | **Gew.-%** |
|---|---|
| Quarternium 18-Hectorite | 1 |
| Ethylen/Propylen/Styrol/Copolymer | 3 |
| Allyl Methacrylate Crosspolymer | 0,5 |
| Hydroxypropylmethylcellulose | 2 |
| Polyvinylalkohol | 5 |
| Polyvinylpyrrolidon | 5 |
| Carbomer (Carbopol 981) | 1 |
| Polyacrylat | 2 |
| Hydroxypropylmethylcellulose | 5 |
| Silica (Aerosil R972) | 2 |
| C_{16 - 38} Alkylhydroxystearoyl-stearat (Kesterwachs K80P) | 2 |
| Caprylic/Capric Triglycerid | 5 |
| Octyldodecanol | 5 |
| Mineralöl | 5 |
| Butylen Glycol Caprylat/Caprat | 6 |
| Ubiquinon | 1 |
| Butyl Methoxydibenzoylmethan | 5 |
| 3-(4-Methylbenzyliden)-campher | 3 |
| Ethylhexyl Methoxycinnamat | 9 |
| Methylen Bis-Benztriazolyl Tetramethylbutylphenol | 8 |
| Phenylbenzimidazol Sulfonsäure | 8 |
| Diethylhexyl Butamido Triazon (UVASORB HEB) | 4 |
| Natrium Maisstärke n-Octenylsuccinat | 1 |
| Chitosan | 2 |
| NaCl | 3 |
| C₁₀₋₃₀ Alkyl Acrylat Crosspolymer (Pemulen TR1) | 2 |
| Methylhydroxyethylcellulose | 4 |
| Wasser | ad 100 |

### Zubereitung 5: Lichtschutzzubereitung

| | **Gew.-%** |
|---|---|
| Ethylcellulose | 5 |
| Polyvinylalkohol | 4 |
| Hydroxyethylcellulose | 5 |
| Dimethicon / Polysilicone-11 | 2 |
| Polyquaternium 37 | 2 |
| Hydroxypropylmethylcellulose | 5 |
| Titandioxid (Eusolex T2000) | 10 |
| Silica (Aerosil R972) | 2 |
| Natrium Maisstärke n-Octenylsuccinat | 1 |
| C_{20 - 40} Alkylstearat (Kesterwachs K82) | 2 |
| Polyisobuten (Rewopal PIB 1000) | 4 |
| Caprylic/Capric Triglycerid | 5 |
| Mineralöl | 5 |
| PVP/ Hecadecene Copolymer | 4 |
| Acetylated Glycol Stearate + Tristearin | 2 |
| Tocopheryl Acetat | 1 |
| Uvinul ® A Plus | 5 |
| Ethylhexyl Methoxycinnamat | |
| Octocrylen | 8 |
| Phenyl Dibenzimidazol Tetrasulfonsäure | 7 |
| Homosalat | 3 |
| Ethylhexylsalicylat | 4 |
| Tapiokastärke | 4 |
| Hyaluronsäure | 4 |
| Magnesiumsulfat | 3 |
| C₁₀₋₃₀ Alkyl Acrylat Crosspolymer (Pemulen TR1) | 2 |
| Wasser | Ad 100 |

### Hilfsstoffabmischungen für Zubereitungen 1 bis 5

| **Angaben in Gew.-%** | **1** | **2** | **3** | **4** | **5** |
|---|---|---|---|---|---|
| Mikrokristalline Cellulose (Avicel PH 102) | 15 | | | | 17,5 |
| Quervernetzte Natrium- Carboxymethylcellulose (Ac-Di-Sol) | 2 | | | 2 | |
| Quervernetztes Polyvinylpyrrolidon | | | 5 | | |
| Natriumhydrogencarbonat | | 2 | | 1 | |
| Feinteilige Kieselsäure (Sipernat 220) | 1 | | 2 | | |
| Zitronensäure | | 4,6 | | 2,3 | |
| Milchzucker | 3 | 5 | 4 | | |
| Saccharose | | 6 | | 4 | |
| Gelatine | | | 1,5 | | |
| Stärke | | 1 | | | |
| Glycerin | | 3 | | | |
| Sorbitol | 2 | | 3 | | |
| Stärke | | | | | |
| Aerosil 200 | | | 1,5 | 1 | |
| Stearinsäure | 1,5 | | 1 | | |
| Magnesiumstearate | | 2 | | 1 | |
| Talkum | 1 | | 2 | | |
| Carbomer (Carbopol 981) | | | | 3 | |
| Polyvinylpyrrolidon | | | | | 4 |
| Xanthan Gummi | 3 | | | | |
| Hydroxyethylcellulose | | | 2,5 | | |
| C₁₀₋₃₀ Alkyl Acrylat Crosspolymer (Pemulen TR1) | | 4 | | | |
| Magnesium Aluminium Silicate (Veegum K) | | | | 1 | |
| Alkohol | | 20 | 15 | 10 | |

## Patentansprüche

1. Kosmetisches und/oder dermatologisches O/W-Emulsionskonzentrat, erhältlich durch Trocknung einer O/W-Emulsion, enthaltend
a) eine Lipidphase in einer Konzentration von 10 bis 85 Gew.-%, bezogen auf das Gesamtgewicht der Zubereitung vor der Trocknung, mit
b) ein oder mehreren öllöslichen Polymeren in einer Gesamtkonzentration von 0,1 bis 50 Gew.-%, bezogen auf das Gesamtgewicht der Zubereitung vor der Trocknung, sowie
c) eine wässrige Phase, mit
d) einem oder mehreren wasserlöslichen Polymeren in einer Gesamtkonzentration von 1 bis 80 Gew.-%, bezogen auf das Gesamtgewicht der Zubereitung vor der Trocknung, sowie
e) ein oder mehrere UV-Lichtschutzfilter und
f) gegebenenfalls weiteren kosmetischen und/oder dermatologischen Wirk-, Hilfs-und Zusatzstoffen,
bis auf einen Wassergehalt von 0,1 bis 20 Gew.-%, bezogen auf das Gesamtgewicht des Emulsionskonzentrates.

2. Kosmetische und/oder dermatologisches O/W-Emulsionskonzentrat nach Anspruch 1, **dadurch gekennzeichnet, dass** die Trocknung durch Walzen- und/oder Kanal-und/oder Sprüh- und/oder Gefriertrocknung erfolgt.

3. Kosmetische und/oder dermatologische Zubereitung enthaltend ein O/W-Emulsionskonzentrat nach mindestens einem der Ansprüche 1 bis 2 und einen oder mehrere Granulier- und/oder Tablettenhilfsstoffe.

4. Kosmetische und/oder dermatologische Zubereitung nach Anspruch 3, **dadurch gekennzeichnet**, das mindestens ein Granulier- und/oder Tablettenhilfsstoff aus den Tabletten-Füllhilfsstoffen, Tabletten-Kompaktierhilfsstoffen, Tabletten-Formtrennmitteln, Tabletten-Bindemitteln, Tabletten-Füllstoffen, Tabletten-Gleitmitten, Tabletten-Schmiermitteln, Tabletten-Fließmitteln, Tabletten-Sprengmittel, Tabletten-Überzügen gewählt werden, insbesondere
- Stärke- und/oder Cellulosederivate,
- hochdisperse Siliziumdioxide,
- Carbonate und/oder Hydrogencarbonate, ganz besonders Natriumhydrogencarbonat und/oder Kaliumhydrogencarbonat,
- bei Raumtemperatur festen Säuren, ganz besonders Zitronensäure, Ascorbinsäure, Milchsäure, Weinsäure,
- vernetztes Polyvinylpyrrolidon,
wobei die Granulier- und/oder Tablettenhilfsstoffe in einer Konzentration von 0,1 bis 60 Gew.-%, bevorzugt in einer Konzentration von 0,1 bis 50 Gew.-% und ganz besonders bevorzugt in einer Konzentration von 0,5 bis 35 Gew.-%, jeweils bezogen auf das Gesamtgewicht der Zubereitung eingesetzt werden.

5. Verfahren zur Herstellung einer kosmetischen und/oder dermatologischen Zubereitung, **dadurch gekennzeichnet, dass** ein O/W-Emulsionskonzentrat nach mindestens einem der Ansprüche 1 bis 2, in einer Mischungsvorrichtung mit wasserlöslichen und/oder leichtflüchtigen Wirk-, Hilfs- und/oder Zusatzstoffen vermischt bzw. abgemischt wird.

6. Verfahren zur Herstellung einer kosmetischen und/oder dermatologischen Zubereitung, **dadurch gekennzeichnet, dass** ein O/W-Emulsionskonzentrat nach mindestens einem der Ansprüche 1 bis 2, mit einem oder mehreren Tablettenhilfsstoffen in einer Mischungsvorrichtung vermischt bzw. abgemischt wird.

7. Verfahren zur Herstellung einer kosmetischen und/oder dermatologischen Zubereitung, **dadurch gekennzeichnet, dass** ein O/W-Emulsionskonzentrat nach mindestens einem der Ansprüche 1 bis 2 oder eine kosmetische und/oder dermatologische Zubereitung nach einem Ansprüche 3 bis 4 oder ein Verfahrensprodukt nach mindestens einem der Ansprüche 5 bis 6, mit Wasser versetzt und unter Einwirkung von Bewegungskräften reemulgiert wird.

8. Verfahren zur Herstellung einer kosmetischen und/oder dermatologischen Zubereitung nach Anspruch 7, **dadurch gekennzeichnet, dass** die Bewegungskräfte durch schütteln, rühren, schleudern, in situ erzeugte Gase entstehen.

9. Verfahren zur Herstellung von Tabletten und/oder Granulaten nach mindestens einem der Ansprüche 3 bis 4, **dadurch gekennzeichnet, dass** eine Zubereitung in einer Presse zu einer oder mehreren Tabletten gepresst oder mittels geeigneter Granuliervorrichtungen granuliert wird.

10. Kosmetikum hergestellt nach mindestens einem Verfahren der Ansprüche 5 bis 9.

11. Kosmetikum nach Anspruch 10, **dadurch gekennzeichnet, dass** die Zubereitung in kompakte Formkörper gepresst werden kann uns sich die kompaktierte Zubereitung mit Hilfe von Wasser reemulgieren lässt.

12. Sonnenschutzpuder enthaltend eine kosmetische und/oder dermatologische Zubereitung nach einem der Ansprüche 1 bis 4.

13. Verwendung einer kosmetische und/oder dermatologische Zubereitung nach einem der Ansprüche 1 bis 4 als Sonnenschutzpuder.

14. Verwendung einer kosmetische und/oder dermatologische Zubereitung nach einem der Ansprüche 1 bis 4 als kosmetisches und/oder kosmetisch dekoratives Puder.
